# EUROPEAN PATENT SPECIFICATION

(11) **EP 1 863 470 B1**
(45) Date of publication and mention of the grant of the patent: **14.10.2009**
(21) Application number: 06720147.5
(22) Date of filing: 01.02.2006
(51) Int. Cl.: A61K 31/192, A61P 15/02

(54) **A METHOD FOR PREVENTING AND/OR TREATING VAGINAL AND VULVAL INFECTIONS**
VERFAHREN ZUR PROPHYLAXE UND/ODER THERAPIE VON INFEKTIONEN DER VAGINA UND VULVA
PROCEDE DE PREVENTION ET/OU DE TRAITEMENT D'INFECTIONS VAGINALES ET VULVAIRES

(30) Priority: 28.03.2005 US 91205
(43) Date of publication of application: 12.12.2007
(73) Proprietor: KIMBERLY-CLARK WORLDWIDE, INC., Neenah, WI 54956 (US)
(72) Inventor: HUANG, Lei, Duluth, GA 30097 (US); YANG, Shu-ping, Alpharetta, GA 30005 (US)
(74) Representative: Leidescher, Thomas
(86) International application number: PCT/US2006/003679
(87) International publication number: WO 2006/104564

(56) References cited:
- WO-A-20/05051365
- WO-A-20/06047590
- DE-C- 876 033
- GB-A- 2 173 400
- PANIZZI L ET AL: "In vitro antimicrobial activity of extracts and isolated constituents of Rubus ulmifolius" JOURNAL OF ETHNOPHARMACOLOGY, vol. 79, no. 2, February 2002 (2002-02), pages 165-168, XP002389601 ISSN: 0378-8741

## Description

The vaginal ecosystem is a finely balanced environment maintained by a complex interaction among vaginal flora. A variety of bacteria, yeasts and other micro-organisms occur naturally in the vagina's environment. *Lactobacillus acidophilus* is the dominant bacteria in a healthy vaginal ecosystem, and it maintains an acidic environment of the vagina through the production of lactic acid. Lactic acid and hydrogen peroxide produced by *Lactobacilli* are toxic to anaerobic bacteria and other pathogenic bacteria in the vagina. The vaginal balance can be upset by external factors such as antibiotics, stress, illness and hormonal changes, and insults that decrease Lactobacilli result in an overgrowth of pathogenic organisms in the vagina.

More than 75% of women will have at least one vaginal infection in their lives, and 50% of these women will have a recurrence of the infection (http://www.stopgettingsick.com/templates/news_template.cfm/1671). It has been reported that in the United States alone, about 13 million women experience vaginal infections each year.

Bacterial vaginosis, the most common vaginal infection, is caused by an overgrowth of a variety of bacterial species, particularly anaerobes. *Gardnerella vaginalis* is the main pathogen in bacterial vaginosis.

Bacterial vaginosis generally shows little or no inflammation of the vaginal epithelium and resembles more of an alteration of the bacterial vaginal environment than a real and proper infection of tissues or epithelium. This pathology is currently treated mainly with antibiotics. Antibiotics, however, also kill the useful bacteria, such as Lactobacilli, resulting in a pH increase in the vaginal environment and increasing the risk of recurrence of the bacterial vaginosis or the development of a different vaginal infection, such as a yeast infection. The antibiotics that are usually administered are metronidazole, clindamycin or ampicillin, which are administered orally. This method of use by the systemic route is frequently accompanied by serious side effects. For example, metronidazole exhibits serious side effects, particularly on the blood and on the central nervous system, so much that in certain types of patients it has been necessary to discontinue the treatment, and authorities in the medical field have recommended that women who use metronidazole should not breast feed (Martindale, The Extra Pharmacopoeia, 29th Edition, 1989, page 667). Clindamycin also exhibits serious side effects, particularly on the gastrointestinal tract, with serious forms of diarrhea and pseudo-membranous colitis that can even lead to the death of the patient (Martindale, pages 198-199).

Trichomonas vaginitis, also known as Trichomoniasis or trich, is one of the most common vaginal infections and this infection is considered to be a sexually transmitted disease. In the United States, it is estimated that more than 2 million women are infected with this each year.

Trichomonas vaginitis causes vulvar itching and an odorous vaginal discharge. It is caused by *Trichomonas vaginalis,* a single-celled protozoan parasite not normally found in the flora of the genitourinary tract. *Trichomonas vaginalis* is a flagellate protozoa that is pear-shaped and about the size of a white blood cell. These motile cells have four flagellae and a single nucleus. Like bacterial vaginosis, this pathology is generally treated with metronidazole.

The yeast *Candida albicans* causes the disease known as candidiasis or "thrush". This is another common vaginal infection, and causes a considerable degree of discomfort. In addition, *Candida* albicans is also the main pathogen that causes vulvitis, a vulval infection. *Candida albicans* is present in most humans as a harmless commensal organism, and thousands of the yeast cells can be present in an individual without any ill effect. Problems arise, however, when a person experiences a loss of normal bacterial flora. *Candida albicans* infection, in severely immune compromised patients, can spread throughout the body and cause deadly systemic infections. Candidiasis is usually treated with fluconazole, but this can have serious side effects and is not recommended for use during pregnancy.

Panizzi et al., In vitro antimicrobial activity of extracts and isolated constituents of Rubus ulmifolius, Journal of Ethnopharmacology, Vol. 79, No. 2, February 2002, pages 165 to 168, describe that the antimicrobial activity on bacteria and fungi of increasing polarity extracts of *Rubus ulmifolius* and that of some isolated constituents, quercetin-3-O-β-D-glucuronide, kaempferol-3-O- β-O-glucuronide, gallic acid, ferulic acid and tiliroside was evaluated. The phenolic and tannins fractions showed a high microbial activity.

From GB 2 173 400 A it is known that dusting powder compositions can contain cyclodextrin, or a polymer thereof and optionally one or more cyclodextrin inclusion complex(es) as vehicle, diluent or excipient in an amount of 0.1 to 99.9 % and biologically active substance(s) and/or odorants and/or coloring agent(s) in an amount of 99.9 to 0.1 %. The dusting powder compositions can therapeutically be used for disinfecting, as well as for mitigating skin inflammations and superficial pains.

German Patent No. 876 033 refers to a method for producing a dry compound comprising durable, acid soluble vitamins, which can particularly be used in animal feed. A wax like substance being solid at ambient temperature having a melting point of at least 45°C is mixed with a substance comprising an acid soluble vitamin and a digestible, surface active substance, as well as optionally an edible oxidizing agent and vegetable flour. The mixture is melted and formed into small, drop- or sphere-shaped particles.

WO 2005/051365 A1 discloses a device for external use designed to improve the location of subcutaneous veins so as to facilitate the practice of venipuncture. The device is based on substances able to highlight the venous system without damaging the skin application area.

From WO 2006/047590 A2 it is known that bacterial vaginosis is treated using methods and compositions that include one or more plant polyphenols as active ingredients. Prefrred routes of administration of contemplated plant polyphenols include topical and oral administration, and particularly preferred plant polyphenols are prepared from green tea. (e.g. Polyphenon E) and/or may include(-)-epigallocatechin gallate, (-)-epicatechin gallate, (-)-epigallocatechin, and (-)-epicatechin.

There is therefore a need for a suitable compound or composition that can treat and/or prevent vaginal and vulval infections without killing the useful bacteria and without the side effects of known treatments.

### SUMMARY OF THE INVENTION

In response to the problems discussed above, it has been found that gallic acid is capable of selectively inhibiting and/or killing the pathogens *Trichomonas vaginalis and Gardnerella vaginalis* without affecting *Lactobacilli growth.* This compound is therefore suitable for use as an active ingredient for treating and/or preventing vaginal and vulval infections caused by bacteria and protozoa, and in particular, infections such as trichomonas vaginitis and bacterial vaginosis.

According to the invention, gallic acid or a pharmaceutically acceptable salt thereof for treating and/or preventing a vaginal infection is described. The gallic acid may be in the form of a solution, a powder and/or a crystalline structure. The gallic acid may be used alone or in a therapeutic amount in a composition, in the form of a foam, a cream, a gel, a jelly, a moisturizer, a spray, a suppository, a vaginal capsule, a vaginal tablet, a vaginal film, a vaginal sponge, a vaginal ovule or any other vaginal health product. The composition may also be applied to a vaginal insert, tampon, wipe or pad. The composition may further include a suitable diluent, excipient and/or auxiliary.

In general, the gallic acid is present in the composition in an amount of from about 0.05 to about 10 percent (grams/100 milliliter (wt/vol)), more preferably in an amount of from about 0.075 to about 5 percent (wt/vol), even more preferably in an amount of from about 0.1 to about 2 percent (wt/vol), and even more preferably in an amount of from about 0.5 to about 1 percent (wt/vol).

The gallic acid or pharmaceutically acceptable salt thereof can be topically administered to a subject in need thereof, so as to inhibit the growth of the pathogenic bacteria, yeast or protozoa without inhibiting the growth of Lactobacillus *acidophilus.*

### BRIEF DESCRIPTION OF THE DRAWINGS

Figure 1 shows the effect of gallic acid on *Trichomonas vaginalis* cell counts after 24 hours;
Figure 2 shows the effect of gallic acid on *Trichomonas vaginalis* cell counts after 48 hours;
Figure 3 shows the effect of gallic acid on *Candida albicans* after 2, 6 and 24 hours; and
Figure 4 shows the effect of gallic acid on *Lactobacillus acidophilus* after 2, 6 and 24 hours.

### DETAILED DESCRIPTION OF THE INVENTION

The invention refers to gallic acid or a pharmaceutically acceptable salt thereof for use in the prevention or treatment of vaginal or vulval infection caused by Trichomonas vaginalis or Gardnerella vaginalis.

Gallic acid or 3,4,5-trihydroxybenzoic acid (C6H2(OH)3CO2 is a naturally occurring polyphenolic antioxidant found in gallnuts. Sumac, green tea, oak bark, grape seed extract and many other plants. It is a colorless crystalline organic acid found both in its free state and as part of the tannin molecule. Gallic acid has hydroxyl groups and a carboxylic acid group in the same molecule, and thus two molecules can react with one another to form an ester, digallic acid. Gallic acid is obtained by the hydrolysis of tannic acid with sulfuric acid. Salts of gallic acid include any physiologically acceptable salt available to one of skill in art. Examples include sodium, calcium or potassium salts of gallic acid.

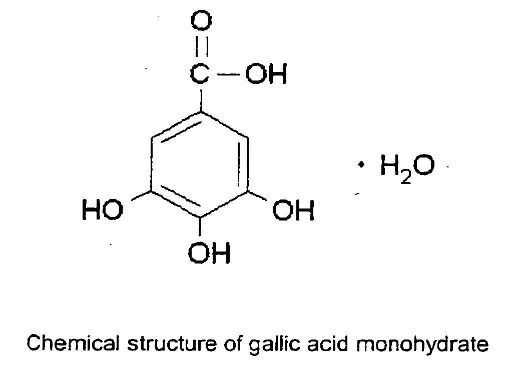

It has previously been shown that gallic acid does not affect the growth of *Lactobacillus acidophilus,* the beneficial flora in the vagina.

The applicant has now surprisingly found that gallic a acid has the effect of selectively inhibiting the parasite *Trichomonas vaginalis and the bacterium Gardnerella vaginalis* while not inhibiting *Lactobacillus acidophilus,* and it is therefore a suitable compound for treating and/or preventing vaginal infections, and in particular, trichomonas vaginitis and bacterial vaginosis.

The gallic acid or pharmaceutically acceptable salt thereof can be used in the form of a solution, powder and/or crystalline structure, either alone or as part of a composition. It is typically administered topically as part of a composition which is in the form of a foam, cream, gel, jelly, moisturizer, spray, suppository, vaginal capsule, vaginal tablet, vaginal film, vaginal sponge, vaginal ovule or any other vaginal health product. The composition optionally also includes suitable diluents, excipients and/or auxiliaries, which are well known in the art.

The composition can be applied to a vaginal insert, tampon, wipe or pad or can be used on its own.

In general, the gallic acid is present in the composition in an amount of from about 0.05 to about 10 percent (grams/100 milliliter (wt/vol)), more preferably in an amount of from about 0.075 to about 5 percent (wt/vol), even more preferably in an amount of from about 0.1 to about 2 percent (wt/vol), and even more preferably in an amount of from about 0.5 to about 1 percent (wt/vol).

The present invention is further described by the following examples. Such examples, however, are not to be construed as limiting in any way either the spirit or scope of the invention.

### EXAMPLES

### Microorganisms and culture media:

A sample of *Trichomonas vaginalis,* the parasite found in trichomonas vaginitis, was obtained from the American Type Culture Collection (ATCC), catalog number 30001. The culture medium was LYI-S-2 medium (ATCC medium 2154).

A sample of *Lactobacillus acidophilus,* a desirable bacterium in the vaginal ecosystem, was also obtained from the American Type Culture Collection (ATCC), catalog number 4354, and was cultured in ATCC medium 416.

A sample of *Gardnerella vaginalis,* the pathogenic bacterium found in bacterial vaginosis, was obtained from the American Type Culture Collection (ATCC), catalog number 14018. The culture medium was ATCC medium 70 and Casman's medium (BD 229010) with 5 percent rabbit blood.

A sample of *Candida albicans,* the yeast found in candidiasis, was also obtained from the American Type Culture Collection (ATCC), catalog number 10231, and was cultured in YM agar (Difco 0712) and YM broth (Difco 0711).

### Example 1: Effect of 1.1 percent gallic acid on the growth of Trichomonas vaginalis

A sterile LYI-S-2 medium was prepared according to the manufacturer's instructions, and the pH of this medium was adjusted to pH 6.0 using 1 N HCl. Gallic acid monohydrate (SIGMA-398225 from Signa Aldrich, USA) was dissolved in the LYI-S-2 medium. The gallic acid monohydrate was found to be only partially water soluble, and the highest concentration of gallic acid that could be obtained in water was 1.1 percent (g/100 milliliter). 0.9 milliliter of the gallic acid/LYI-S-2 solution or culture medium only (as control) was added into different culture tubes.

0.1 milliliter of *Trichomonas vaginalis* culture suspension, at a concentration of 1x10⁶/milliliter, was added to each of the culture tubes, which were then incubated at 35 degrees Celsius on a 15 degree horizontal slant.

The viable *Trichomonas vaginalis* cells in each tube were counted under a microscope after 24 hours.

This procedure was repeated twice.

The results in Table 1 show that gallic acid at a concentration of 1.1 percent completely reduced the *Trichomonas vaginalis* cell count after 24 hours and no live *Trichomonas vaginalis* cells were observed in the treatment group. In contrast, between 0.9 and1 million live *Trichomonas vaginalis* cells were counted in the control group.

**Table 1: Number of Trichomonas cells with or without gallic acid treatment**

| | Repeat 1 | Repeat 2 |
|---|---|---|
| Culture medium only (control) | 900 000 | 1 000 000 |
| Gallic acid (1.1 percent) | 0 | 0 |

### Example 2: Effect of various concentrations of gallic acid on the growth of Trichomonas vaginalis

Essentially the same procedure was performed as described in Example 1, but this time differing concentrations of gallic acid were tested on samples of *Trichomonas vaginalis.* The gallic acid concentrations that were tested were: 0.11 percent, 0.66 percent and 1.1 percent. More samples in each group were included (n=4), and the effects of the gallic acid over a longer time period were also observed, i.e. after 24 and 48 hours.

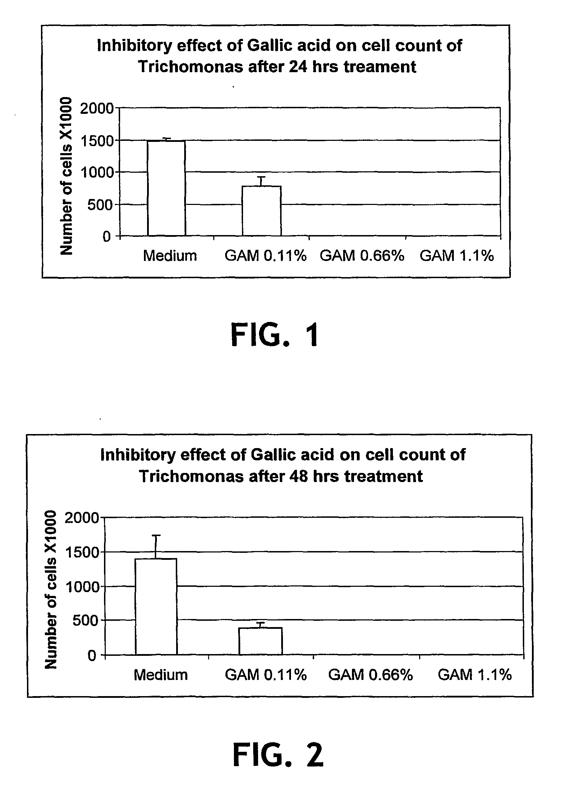

As shown in Figures 1 and 2, gallic acid at a concentration of 0.11 percent showed a 50 to 70 percent inhibitory effect on *Trichomonas vaginalis* cell counts after 24 and 48 hours, respectively, compared to the control group (medium only). No live *Trichomonas vaginalis* cells were observed in the samples that were treated with gallic acid concentrations of 0.66 or 1.1 percent, either after 24 or 48 hours.

### Example 3: Effect of Gallic acid on the growth of Candida albicans and Lactobacillus acidophilus - zone-of-inhibition test

A microorganism culture of 10⁵ cfu (colony forming units)/milliliter in a 1 x phosphate buffered saline (PBS) solution (diluted from 10X PBS LIQUID CONCENTRATE from VWR Cat. No. EM-6507] was prepared for each of *Candida albicans* and *Lactobacillus acidophilus.* One milliliter of each solution was plated on proper agar plates, depending on which microorganism was being tested. The agar plates were incubated at 35 degrees Celsius for four hours. Three 4 millimeter diameter wells were then punched into each agar plate. A test sample of 10 mg/ml gallic acid in sterilized 2-N-morpholino ethane sulfonic (MES, pH=4.7) buffer (0.1 M 2-[morpholino]-ethanesulfonic acid, 0.9 percent NaCl, pH 4.7, prepared from BupH™ MES Buffer Saline Pack from Cat. No. 28390, Pierce Biotechnology, Inc., Rockford, IL) was added to one well of each plate. Into each of the other two wells were added MES buffer and 0.5 percent Benzyl Quats (diluted from BARDAC® 205M, from Lonza Inc., Fair Lawn, New Jersey) as negative and positive controls, respectively. The plates were incubated overnight at 35 degrees Celsius. The presence of a zone of microorganism inhibition was measured the following day for *Candida albicans* and *Lactobacillus acidophilus* activity, respectively.

As shown in Table 2, gallic acid at a concentration of 10 mg/ml selectively inhibited *Candida albicans,* while it did not affect the growth of *Lactobacillus acidophilus.* The positive control, 0.5 percent Benzyl Quats, inhibited the growth of both microorganisms, while MES buffer itself had no effect on either of the two microorganisms.

**Table 2: Effect of gallic acid on Candida albicans and Lactobacillus acidophilus with zone of inhibition test, n = 2.**

| Tested Compounds/Polymers | *Candida albicans* | *Lactobacillus acidophilus* |
|---|---|---|
| 10 milligram/ml gallic acid | 5 millimeter | 0 millimeter |
| 0.5 percent Benzyl Quats | 5 millimeter | 7 millimeter |
| MES buffer | 0 millimeter | 0 millimeter |

### Example 4: Effect of gallic acid on the growth of Candida albicans and Lactobacillus acidophilus - inhibition tests in solution by measuring optical density

Test compounds of *Candida albicans* and *Lactobacillus acidophilus* were dissolved in culture media to form a suspension. Control and gallic acid solutions (0.9 milliliters) were filtered and added into separate culture tubes, and to these were added 0.1 milliliter of either the *Candida albicans* or *Lactobacillus acidophilus* suspension at a concentration of around 10⁶ cfu/milliliter. The culture tubes were then incubated overnight at 37 degrees Celsius, whereafter the optical density was measured at 2, 4, 6 and 24 hours at 590 nanometers, by pippeting 100 microliters of the control or sample solutions into 96-well microplates, and then using a Molecular Devices of Sunnyvale, CA ThermoMax Microplate Reader to obtain the optical density readings at 590 nm wavelengths.

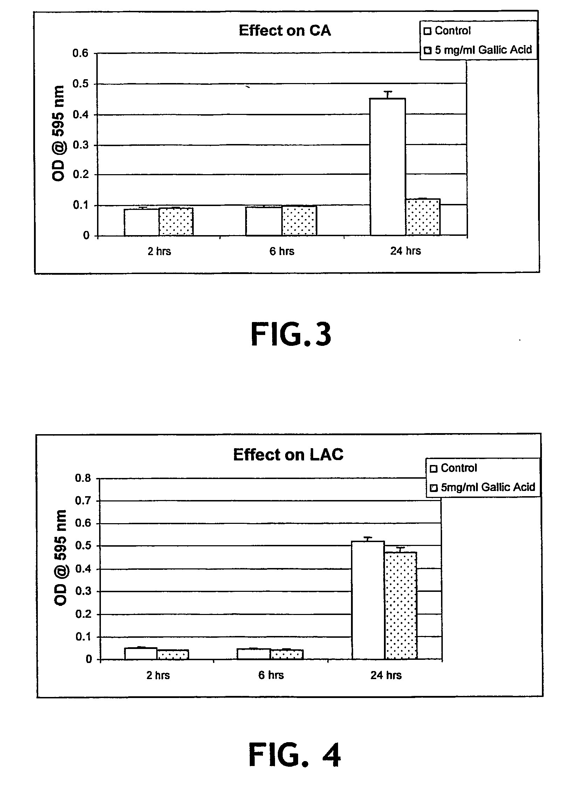

Gallic acid at a concentration of 5 milligram/milliliter was shown to significantly inhibit the growth of *Candida albicans* after 24 hours of treatment (Figure 3).

In contrast to the profound inhibition on the growth of *Candida albicans,* gallic acid at the same concentration did not show any significant inhibition on the growth of *Lactobacillus acidophilus* after 24 hours (Figure 4).

These optical density results were consistent with the zone-of-inhibition results of Example 3.

### Example 5: Effect of gallic acid on the growth of Gardnerella vaginalis - inhibition test in solution by plate count

A test compound of *Gardnerella vaginalis* was prepared in culture media to form a suspension. Control and gallic acid solutions (0.9 milliliters; 7.5 milligram gallic acid /milliliter) were filtered and added into separate culture tubes, and to these were added 0.1 milliliter of the *Gardnerella vaginalis* suspension at a concentration of around 10⁶ cfu/milliliter. The culture tubes were incubated at 37 degrees Celsius for 24 hours.

The samples in the culture tubes were then diluted at 1, 10 and 100 times, and 100 microliters of each dilution was plated onto agar plates with WASP (Whitely Automatic Spiral Plate) spiral plating equipment from Don Whitely Scientific Limited, USA. The plates were incubated overnight at 35 degrees Celsius, and the colonies were counted on each plate by either ProtoCol® from Synbiosis, Frederick, MD, USA Whitely Scientific Limited, USA or by hand count.

After 24 hours of treatment, gallic acid showed significant inhibition (over 99.98 percent) on the growth of *Gardnerella vaginalis* compared to a control group (Table 3).

**Table 3: Effect of gallic acid on Gardnerella vaginalis after 24 hours treatment, n=4**

| | Negative control | 7.5 mg/ml gallic acid |
|---|---|---|
| Average | 1.81 E+08 | *3.43E+04 |
| STDEV | 6.36E+06 | 2.12E+03 |

| | | |
|---|---|---|
| * represents p< 0.05 compared to negative control group | | |

The results of the above examples clearly demonstrate that different concentrations of gallic acid are able to effectively inhibit *Trichomonas vaginilis, Gardnerella vaginalis* and *Candida albicans.* As gallic acid has also been shown not to inhibit the growth of *Lactobacillus acidophilus,* and as it is a naturally occurring, safe compound which is also cost-effective, it is ideally suited to be formulated into vaginal health products, such as tampons, pads, wipes, vaginal moisturizers, sprays, gels and so forth for preventing and/or treating vaginal infections such as trichomonas vaginitis, bacterial vaginosis and candidiasis.

## Claims

1. Gallic acid or a pharmaceutically acceptable salt thereof for use in the prevention or treatment of vaginal or vulval infection caused by *Trichomonas vaginalis* or *Gardnerella vaginalis.*

2. Gallic acid or a pharmaceutically acceptable salt thereof according to claim 1, which is used in the form of a solution, powder and/or crystalline structure.

3. Gallic acid or a pharmaceutically acceptable salt thereof according to claim 2, which is used alone.

4. Gallic acid or a pharmaceutically acceptable salt thereof according to claim 2 which is a part of a composition.

5. Gallic acid or a pharmaceutically acceptable salt thereof according to claim 4, wherein the composition is in the form of a foam, cream, gel, jelly, moisturizer, spray, suppository, vaginal capsule, vaginal tablet, vaginal film, vaginal sponge, or vaginal ovule.

6. Gallic acid or a pharmaceutically acceptable salt thereof according to claim 4 or 5, wherein the composition further comprises diluents, excipients and /or auxiliaries.

7. Gallic acid or a pharmaceutically acceptable salt thereof according to one of claims 4 to 6, wherein the composition is applied to a vaginal insert, tampon, wipe or pad.

8. Gallic acid or a pharmaceutically acceptable salt thereof according to one of claims 4 to 7, wherein the gallic acid is present in the composition in amount of from 0.05 to 10 percent (grams/100 milliliter (wt/vol)).

9. Gallic acid or a pharmaceutically acceptable salt thereof according to one of claims 4 to 7, wherein the gallic acid is present in the composition in amount of from 0.075 to 5 percent (grams/100 milliliter (wt/vol)).

10. Gallic acid or a pharmaceutically acceptable salt thereof according to one of claims 4 to 7, wherein the gallic acid is present in the composition in amount of from 0.1 to 2 percent (grams/100 milliliter (wt/vol)).

11. Gallic acid or a pharmaceutically acceptable salt thereof according to one of claims 4 to 7, wherein the gallic acid is present in the composition in amount of from 0.5 to 1 percent (grams/100 milliliter (wt/vol)).

## Patentansprüche

1. Gallussäure oder ein pharmazeutisch akzeptables Salz davon zur Verwendung in der Vermeidung oder Behandlung von vaginalen oder die Vulva betreffenden Infektionen, welche durch *Trichomonas vaginalis* oder *Gardnerella vaginalis* verursacht werden.

2. Gallussäure oder ein pharmazeutisch akzeptables Salz davon gemäß Anspruch 1, welche/welches in Form einer Lösung, eines Pulvers und/oder einer kristallinen Struktur verwendet wird.

3. Gallussäure oder ein pharmazeutisch akzeptables Salz davon gemäß Anspruch 2, welche/welches alleine verwendet wird.

4. Gallussäure oder ein pharmazeutisch akzeptables Salz davon gemäß Anspruch 2, welche/welches ein Teil einer Zusammensetzung ist.

5. Gallussäure oder ein pharmazeutisch akzeptables Salz davon gemäß Anspruch 4, wobei die Zusammensetzung in Form eines Schaums, einer Creme, eines Gels, eine Gelees, einer Feuchtigkeitscreme, eines Sprays, eines Zäpfchens, einer Vaginal-Kapsel, einer Vaginal-Tablette, einem Vaginal-Film, einem Vaginal-Schwamm oder einer Vaginal-Ovula vorliegt.

6. Gallussäure oder ein pharmazeutisch akzeptables Salz davon gemäß Anspruch 4 oder 5, wobei die Zusammensetzung des Weiteren Verdünnungsmittel, Arzneiträger und/oder Hilfsstoffe umfasst.

7. Gallussäure oder ein pharmazeutisch akzeptables Salz davon gemäß einem der Ansprüche 4 bis 6, wobei die Zusammensetzung auf eine vaginale Einlage, einen Tampon, ein Tuch oder ein Pad aufgebracht ist.

8. Gallussäure oder ein pharmazeutisch akzeptables Salz davon gemäß einem der Ansprüche 4 bis 7, wobei die Gallussäure in der Zusammensetzung in einer Menge von 0,05 bis 10 Prozent (Gramm/100 Mililiter (Gew./Vol.)) vorhanden ist.

9. Gallussäure oder ein pharmazeutisch akzeptables Salz davon gemäß einem der Ansprüche 4 bis 7, wobei die Gallussäure in der Zusammensetzung in einer Menge von 0,075 bis 5 Prozent (Gramm/100 Mililiter (Gew./Vol.)) vorhanden ist.

10. Gallussäure oder ein pharmazeutisch akzeptables Salz davon gemäß einem der Ansprüche 4 bis 7, wobei die Gallussäure in der Zusammensetzung in einer Menge von 0,1 bis 2 Prozent (Gramm/100 Mililiter (Gew./Vol.)) vorhanden ist.

11. Gallussäure oder ein pharmazeutisch akzeptables Salz davon gemäß einem der Ansprüche 4 bis 7, wobei die Gallussäure in der Zusammensetzung in einer Menge von 0,5 bis 1 Prozent (Gramm/100 Mililiter (Gew./Vol.)) vorhanden ist.

## Revendications

1. Acide gallique ou sel pharmaceutiquement acceptable de celui-ci, pour utilisation dans la prévention ou le traitement d'une infection vaginale ou vulvaire causée par *Trichomonas vaginalis* ou *Gardnerella vaginalis.*

2. Acide gallique ou sel pharmaceutiquement acceptable de celui-ci selon la revendication 1, utilisé sous la forme d'une solution, d'une poudre et/ou d'une structure cristalline.

3. Acide gallique ou sel pharmaceutiquement acceptable de celui-ci selon la revendication 2, utilisé seul.

4. Acide gallique ou sel pharmaceutiquement acceptable de celui-ci selon la revendication 2, faisant partie d'une composition.

5. Acide gallique ou sel pharmaceutiquement acceptable de celui-ci selon la revendication 4, dans lequel la composition est sous la forme d'une mousse, d'une crème, d'un gel, d'une gelée, d'une forme hydratante, d'un nébulisat, d'un suppositoire, d'une gélule vaginale, d'un comprimé vaginal, d'un film vaginal, d'une éponge vaginale ou d'un ovule vaginal.

6. Acide gallique ou sel pharmaceutiquement acceptable de celui-ci selon la revendication 4 ou 5, dans lequel la composition comprend, en outre, des diluants, excipients et/ou agents auxiliaires.

7. Acide gallique ou sel pharmaceutiquement acceptable de celui-ci selon l'une quelconque des revendications 4 à 6, dans lequel la composition est appliquée sous la forme d'un insert, d'un tampon, d'une lingette ou d'un matériau comprimé vaginal.

8. Acide gallique ou sel pharmaceutiquement acceptable de celui-ci selon l'une quelconque des revendications 4 à 7, dans lequel l'acide gallique est présent dans la composition en une quantité comprise entre 0,05 et 10 pour cent [grammes/100 millilitres (poids:volume)].

9. Acide gallique ou sel pharmaceutiquement acceptable de celui-ci selon l'une quelconque des revendications 4 à 7, dans lequel l'acide gallique est présent dans la composition en une quantité comprise entre 0,075 et 5 pour cent [grammes/100 millilitres (poids:volume)].

10. Acide gallique ou sel pharmaceutiquement acceptable de celui-ci selon l'une quelconque des revendications 4 à 7, dans lequel l'acide gallique est présent dans la composition en une quantité comprise entre 0,1 et 2 pour cent [grammes/100 millilitres (poids:volume)].

11. Acide gallique ou sel pharmaceutiquement acceptable de celui-ci selon l'une quelconque des revendications 4 à 7, dans lequel l'acide gallique est présent dans la composition en une quantité comprise entre 0,5 et 5 pour cent [grammes/100 millilitres (poids:volume)].
